# EUROPÄISCHE PATENTSCHRIFT

(11) **EP 0 464 973 B1**
(45) Veröffentlichungstag und Bekanntmachung des Hinweises auf die Patenterteilung: **03.01.1996**
(21) Anmeldenummer: 91250163.2
(22) Anmeldetag: 20.06.1991
(51) Int. Cl.: A61M 1/10

(54) **Medizinische Vorrichtung zur Erzeugung eines alternierenden Volumenstroms für den Antrieb von implantierbaren Blutpumpen**
Medical device generating an alternating flow for driving implantable blood pumps
Dispositif médical produisant un courant volumétrique alternatif pour la commande de pompes implantables pour le sang

(30) Priorität: 25.06.1990 DE 4020120
(43) Veröffentlichungstag der Anmeldung: 08.01.1992
(73) Patentinhaber: Affeld, Klaus, Prof. Dr. Ing., D-10629 Berlin (DE)
(72) Erfinder: Affeld, Klaus, Prof. Dr. Ing., D-10629 Berlin (DE)
(74) Vertreter: Linser, Heinz

(56) Entgegenhaltungen:
- CH-A- 143 798
- DE-A- 1 528 798
- DE-A- 2 107 000
- US-A- 4 382 199
- US-A- 4 888 011
- BIOMEDIZINISCHE TECHNIK Bd. 19, Nr. 6, 1974, BERLIN Seiten 217 - 224; G. GEISSELBRECHT ET AL: 'EIN INKORPORIERBARES ANTRIEBSSYSTEM FÜR BLUTPUMPEN ZUR ENTLASTUNG DES LINKEN HERZENS '

## Beschreibung

Die Erfindung betrifft eine medizinische Vorrichtung zur Erzeugung eines alternierenden Volumenstroms für den Antrieb implantierbarer Blutpumpen.

Es sind Vorrichtungen, im folgenden als Blutpumpenantriebe bezeichnet, bekannt, die einen alternierenden Volumenstrom eines Übertragungsfluids durch die Umkehr der Drehrichtung einer Axialpumpe erzeugen. Diese Umkehr des Drehsinns hat den Nachteil eines zusätzlichen Energieverbrauchs und zusätzlicher Massenkräfte. Auch werden die Lager des Laufrads durch die Umkehr belastet, da ein Schmierfilm bei jedem Nulldurchgang zerreißt und ein Verschleiß auftritt, was besonders bei der Anwendung als vollimplantierbarer Antrieb ein Nachteil ist, weil dadurch die Zuverlässigkeit verringert wird.

Weiterhin sind Blutpumpenantriebe bekannt, die den kontinuierlichen Strom einer axialen oder radialen Kreiselpumpe durch ein spezielles Schaltventil so umsteuern, daß beim Verbraucher ein alternierender Volumenstrom des Übertragungsfluids entsteht. Dieses Schaltventil stellt jedoch ein weiteres bewegtes Element dar und bedeutet damit eine Verringerung der Zuverlässigkeit. Auch wird das Bauvolumen dadurch vergrößert.

Aus der Druckschrift "Biomedizinische Technik, Band 19/1974, Nr. 6" ist ein implantierbarer elektrohydraulischer Antrieb für Blutpumpen bekannt, welcher eine physiologische Pulsform erzeugt. Für die Wirkungsweise dieses Antriebs ist ein periodischer Betrieb einer Kreiselpumpe charakteristisch. Während der Pumpphase fördert die Kreiselpumpe die Antriebsflüssigkeit aus dem hydraulischen Speicher in die Blutpumpe. Dadurch wird eine mechanische Feder im Speicher gespannt und damit potentielle Energie in der Feder gespeichert. Während der Saugphase ist die Kreiselpumpe ausgeschaltet. Durch die gespeicherte potentielle Energie wird die Antriebsflüssigkeit aus der Blutpumpe durch die stillstehende Kreiselpumpe hindurch in den Speicher zurückgepumpt.

Besonders nachteilig ist es, daß ein weiteres mechanisches Federelement mit einem komplexen Bewegungsablauf vorhanden ist, welches sich kaum hysteresfrei herstellen läßt und damit mechanische Energie in Wärme umsätzt. Ferner ist es nachteilig eine Kreiselpumpe intermittierend zu verwenden, da durch die periodische Verzögerung und Beschleunigung der rotierenden Elemente der Kreiselpumpe weitere Energieverluste auftreten.

Der Erfindung liegt daher die Aufgabe zugrunde, die oben erwähnten Nachteile der bisherigen Lösungen zu vermeiden und die Aufgabe mit einem Minimum an mechanischen Elementen energiesparend zu lösen.

Dies wird gemäß der Erfindung dadurch erreicht, daß die Vorrichtung ein Gehäuse mit zwei Auslässen aufweist, in dem ein radial wirkendes doppelflutiges Kreiselpumpenlaufrad in axialer Richtung verschiebbar angeordnet ist, wodurch die Förderung eines Übertragungsfluids von dem einen Auslaß zum anderen Auslaß umschaltbar ist, und so ohne Umkehrung des Drehsinns ein alternierender Förderstrom für den Antrieb einer oder zweier Blutpumpen erreichbar ist, wobei die Vorrichtung zu beiden Seiten des Kreiselpumpenlaufrades je ein Element aufweist, mit dem die Einlaßöffnung auf jeweils einer Seite des Kreiselpumpenlaufrades abdeckbar und damit verschließbar ist.

Mit der Vorrichtung nach der Erfindung wird der Förderstrom in einem speziellen Gehäuse in eine andere Strombahn geleitet. Der Zustrom zum Kreiselpumpenlaufrad wechselt dabei von der einen zur anderen Seite. Wird das Kreiselpumpenlaufrad wieder axial in seine ursprüngliche Position zurückgeschoben, so tritt wiederum eine Umkehrung der Förderrichtung auf. Ohne eine Umkehrung des Drehsinns oder andere konstruktive Maßnahmen wird so eine Umkehrung der Förderrichtung erreicht, also ein alternierender Förderstrom.

Die mit der Erfindung erzielten Vorteile bestehen insbesondere darin, daß das radial durchströmte Kreiselpumpenlaufrad zur Erzeugung des alternierenden Vorlumenstroms in seiner Drehrichtung nicht umgekehrt oder angehalten zu werden braucht. Damit können hydrodynamisch wirkende Gleitlager eingesetzt werden, bei denen bei richtiger Auslegung ein Kontakt der Lagermaterialien nicht auftritt und die damit eine unbegrenzte Lebensdauer haben können. Bei einer Umkehrung der Drehrichtung, die bei manchen der bekannten Konstruktionen erforderlich ist, können derartige Lager nicht eingesetzt werden, da bei einem Nulldurchgang der Drehzahl der Schmierfilm zerreißt und Verschleiß auftritt. Für Anwendungen wie bei einer im Körper implantierten Blutpumpe, bei denen eine hohe Zuverlässigkeit im Vordergrund steht, ist dies ein entscheidender Vorteil. Der alternierende Förderstrom wird also durch nur ein einziges bewegliches Bauelement erzeugt und damit ist die größtmögliche Einfachheit erreicht.

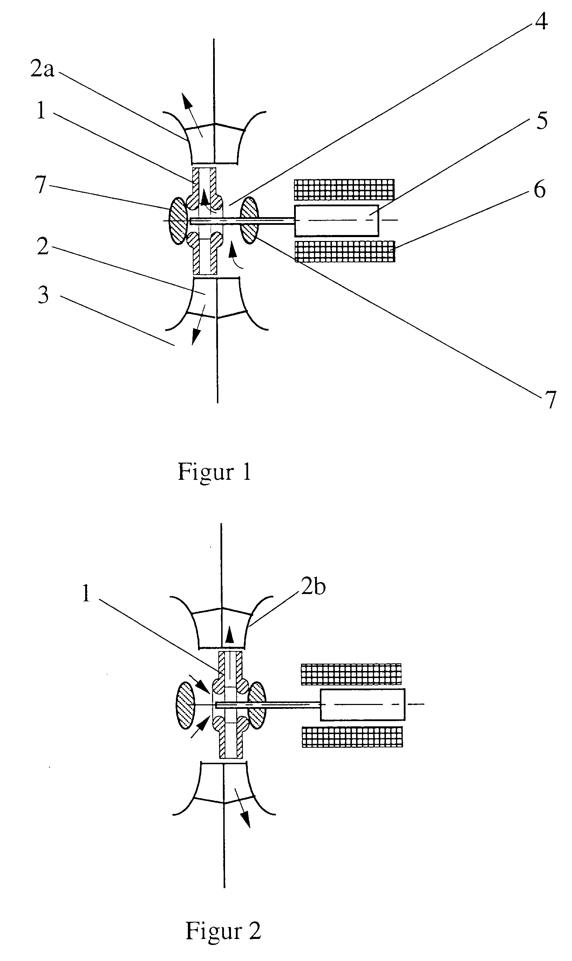

Fig. 1 zeigt eine Ausbildung der Vorrichtung, bei der das Kreiselpumpenlaufrad 1 über einen Diffusor 2 in die Abstrombahn 3 fördert. Der Zustrom erfolgt durch die Zustromöffnung 4. Angetrieben wird das Kreiselpumpenlaufrad durch den Anker 5, der durch den Stator 6 den Drehimpuls erhält. Gehäusefeste Elemente 7 decken jeweils eine Zustromöffnung 2a oder 2b des doppelflutigen Kreiselpumpenlaufrades 1 ab.

Fig. 2 zeigt die gleiche Ausbildung der Vorrichtung bei axialer Verschiebung des Kreiselpumpenlaufrades 1 um eine Schaufelbreite, wodurch eine Umkehr der Förderrichtung erreicht wird, die durch die Pfeile gekennzeichnet ist.

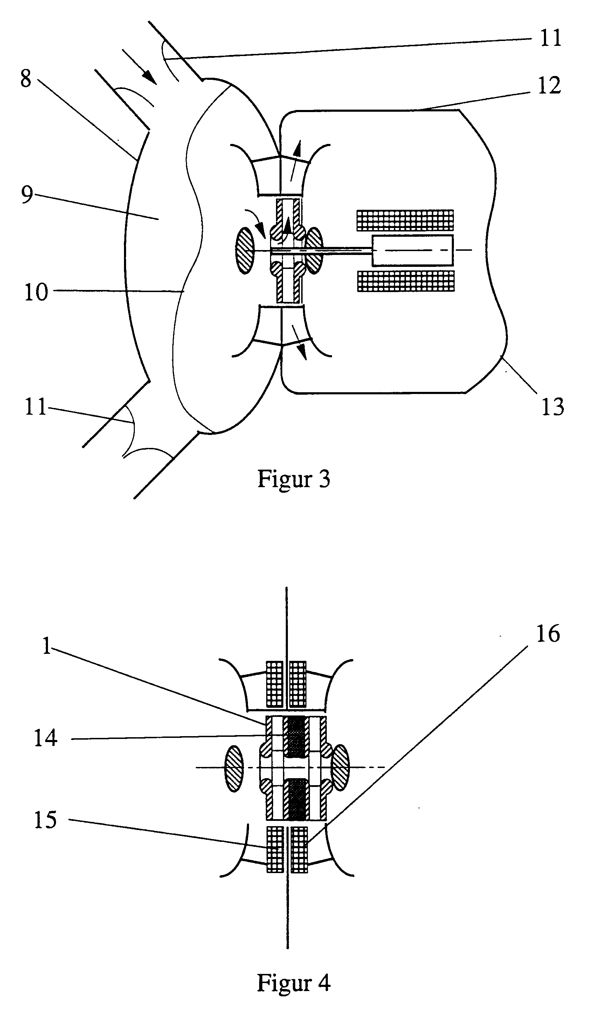

Fig. 3 zeigt eine weitere Ausbildung der Vorrichtung, zusätzlich eingezeichnet ist hier die Verdrängerpumpe 8, die das eigentliche Förderfluid 9, nämlich das Blut, fördert. Die Förderleistung des Kreiselpumpenlaufrade 1 wird dabei über eine verschiebliche Wandung, etwa eine flexible Membran 10, auf das Förderfluid 9 übertragen. Zwei Ventile 11 ermöglichen die gerichtete Förderung des Förderfluids 9. Das Übertragungsfluid, das vom Kreiselpumpenlaufrad 1 in die Verdrängerpumpe 8 hinein- bzw. hinausgepumpt wird, wird dabei aus dem Gefäß 12 heraus- bzw. hineingepumpt. Den erforderlichen Ausgleich des Volumens ermöglicht eine flexible Membran 13, die auch als die Membran einer zweiten Verdrängerpumpe 8 wirken kann.

Fig. 4 zeigt eine weitere Ausbildung der Vorrichtung, bei der der Anker 14 des elektromotorischen Antriebs in das Kreiselpumpenlaufrad 1 integriert ist, das hier aus Raumgründen zwei Kanäle besitzt. Die Statoren 15 und 16 bewirken dabei eine Übertragung des Drehimpulses und können auch gleichzeitig die axiale Verschiebung bewirken.

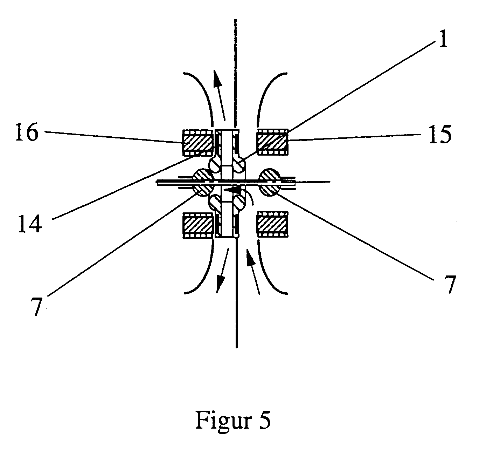

Fig. 5 zeigt eine weitere Ausbildung der Vorrichtung, bei der der Anker des elektromotorischen Antriebs ebenfalls in das Kreiselpumpenlaufrad 1 integriert ist, hier wirkt das Magnetfeld der Statoren 15 und 16 seitlich auf den Anker 14, der hier doppelt vorhanden ist. Die Verschiebung und das Halten des Kreiselpumpenlaufrades 1 in den Endpositionen wird ebenfalls durch das antreibende Feld bewirkt, kann aber ebenfalls durch Magnete in den Elementen 7 bewirkt oder unterstützt werden.

Die Erfindung kann auch mit einer oder mehreren Blutpumpen als künstliches Herz oder als Herzunterstützungspumpe Anwendung finden.

## Patentansprüche

1. Medizinische Vorrichtung zur Erzeugung eines alternierenden Volumenstroms für den Antrieb implantierbarer Blutpumpen **dadurch gekennzeichnet, daß** die Vorrichtung ein Gehäuse mit zwei Auslässen (2a,2b) aufweist, in dem ein radial wirkendes doppelflutiges Kreiselpumpenlaufrad (1) in axialer Richtung verschiebbar angeordnet ist, wodurch die Förderung eines Übertragungsfluids von dem einen Auslaß (2a) zum anderen Auslaß (2b) umschaltbar ist, und so ohne Umkehrung des Drehsinns ein alternierender Förderstrom für den Antrieb einer oder zweier Blutpumpen erreichbar ist, und daß die Vorrichtung zu beiden Seiten des Kreiselpumpenlaufrades (1) je ein Element (7) aufweist, mit dem die Einlaßöffnung (4) auf jeweils einer Seite des Kreiselpumpenlaufrades (1) abdeckbar und damit verschließbar ist.

2. Vorrichtung nach Anspruch 1, **dadurch gekennzeichnet, daß** sie zum Antrieb des Kreiselpumpenlaufrades (1) einen im Kreiselpumpenlaufrad (1) integrierten Elektromotor (5,6) aufweist.

3. Vorrichtung nach Anspruch 1 oder 2, **dadurch gekennzeichnet, daß** die axiale Verschiebung des Kreiselpumpenlaufrades (1) durch die Anziehungskraft des antreibenden Feldes erreichbar ist.

4. Vorrichtung nach einem der vorgenannten Ansprüche, **dadurch gekennzeichnet, daß** die Vorrichtung spezielle Magnete aufweist, durch deren Anziehungskraft die axiale Verschiebung des Kreiselpumpenlaufrades (1) und das Halten des Kreiselpumpenlaufrades (1) in den Endlagen erreichbar ist.

## Claims

1. Medical device for producing an alternating mass flow for driving implantable blood pumps, characterised in that the device has a housing with two outlets (2a, 2b) in which a radially acting dual-flow centrifugal pump impeller (1) is arranged so as to be slidable in an axial direction, by means of which delivery of a transmission fluid can be switched from the one outlet (2a) to the other outlet (2b) and thus generate an alternating delivery flow for the drive of one or two blood pumps and in that the device has respectively on each side of the centrifugal pump impeller (1) an element (7) by means of which the inlet orifice (4) on a respective side of the centrifugal pump impeller (1) can be covered and thereby closed off.

2. Device as claimed in claim 1, characterised in that it has an electric motor (5, 6) integrated in the centrifugal pump impeller (1) for driving the centrifugal pump impeller (1).

3. Device as claimed in claim 1 or 2, characterised in that the axial sliding action of the centrifugal pump impeller (1) can be obtained by the force of attraction of the induced field.

4. Device as claimed in one of the previous claims, characterised in that the device has special magnets, whose force of attraction operates the axial sliding of the centrifugal pump impeller (1) and retention of the centrifugal pump impeller (1) in the end positions.

## Revendications

1. Dispositif médical pour produire un flux volumique pour l'entraînement d'une pompe sanguine implantable, caractérisé en ce que le dispositif présente un boîtier à deux sorties (2a, 2b), dans lequel un rotor de pompe centrifuge (1) à double flux, travaillant radialement, est disposé de manière à pouvoir être déplacé dans une direction axiale, grâce à quoi le refoulement d'un fluide de transfert peut être commuté d'une sortie (2a) à l'autre sortie (2b), et un courant de refoulement alternatif peut ainsi être obtenu, sans inversion du sens de rotation, pour l'entraînement d'une ou de deux pompes sanguines, et en ce que le dispositif présente de chaque côté du rotor de pompe centrifuge (1) un élément (7) au moyen duquel l'ouverture d'entrée (4) d'un côte du rotor de pompe centrifuge (1) peut chaque fois être recouverte et donc fermée.

2. Dispositif selon la revendication 1, caractérisé en ce qu'il présente un moteur électrique (5, 6) intégré au rotor de pompe centrifuge (1), pour l'entraînement du rotor de pompe centrifuge (1).

3. Dispositif selon la revendication 1 ou 2, caractérisé en ce que le déplacement axial du rotor de pompe centrifuge (1) peut être obtenu par la force d'attraction du champ moteur.

4. Dispositif selon l'une quelconque des revendications précédentes, caractérisé en ce que le dispositif présente des aimants spéciaux, dont la force d'attraction permet d'obtenir le déplacement axial du rotor de pompe centrifuge (1) et le maintien du rotor de pompe centrifuge (1) dans les positions d'extrémité.
